(19) Europäisches Patentamt / European Patent Office / Office européen des brevets

(11) **EP 2 839 782 A1**

(12) **EUROPEAN PATENT APPLICATION**
published in accordance with Art. 153(4) EPC

(43) Date of publication:
**25.02.2015 Bulletin 2015/09**

(21) Application number: **12874860.5**

(22) Date of filing: **21.08.2012**

(51) Int Cl.:
*A61B 6/00* (2006.01)    *G01N 9/36* (2006.01)
*G01N 23/083* (2006.01)

(86) International application number:
**PCT/RU2012/000688**

(87) International publication number:
**WO 2013/157983 (24.10.2013 Gazette 2013/43)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **19.04.2012 RU 2012115655**

(71) Applicant: **Obschestvo Ogranichennoj Otvetstvennost'Ju "Parafarm"**
**440026 Penza (RU)**

(72) Inventors:
• **STRUKOV, Villorij Ivanovich**
  **Penza Region**
  **Penza 440011 (RU)**
• **JHONES, Olga**
  **Fort Wors, Texas 76107 (US)**
• **KRUTIAKOV, Evgenij Nikolaevich**
  **Penza 440062 (RU)**
• **ELISTRATOV, Konstantin Gennad'evich**
  **Penza Region**
  **Penza 440061 (RU)**

(74) Representative: **Wablat Lange Karthaus Anwaltssozietät**
**Potsdamer Chaussee 48**
**14129 Berlin (DE)**

(54) **METHOD FOR DIAGNOSING OSTEOPOROSIS BY A METHOD FOR DEFINING THE DYNAMICS OF CLOSING CAVITY FORMATIONS IN ORDER TO ASSESS THE EFFECTIVENESS OF USING VARIOUS OSTEOPROTECTORS**

(57)    The method for diagnosing osteoporosis, using a method for determining the dynamics of closure of cavities to evaluate the effectiveness of the application of various bone protectors, relates to the field of medicine, for the diagnosis of osteoporosis and measurement of the effectiveness of therapeutic and prophylactic treatment of conditions associated with osteoporosis. The method is characterised by laboratory, histological and radiological methods of examination (standard radiography) and by measuring bone mineral density using the radiological absorptive method on osteometers, into which an additional diagnostic criterion is introduced, namely the measurement of cavities by specially adjusting them to measure cavities in trabecular bone sections using the radiological absorptive method and in addition, distilled water is introduced into special incubation apparatuses around the limb bones of the hands and feet which are being examined. The problem being addressed by the claimed invention is to objectively evaluate the effectiveness of a preparation (preparations) (bone protector) and measure cavities in trabecular bone sections. It proposes to improve the diagnostic capabilities of computerised osteometers by measuring cavities caused by osteoporosis and determine the severity of the disease not only on the basis of mineral density, but also on the basis of the presence of cavities in trabecular bone sections.

EP 2 839 782 A1

**Description**

This invention relates to the field of medicine, for the diagnosis of osteoporosis and measurement of the effectiveness of therapeutic and prophylactic treatment of conditions associated with osteoporosis.

Osteoporosis (hereinafter referred to as OP) is a metabolic skeletal disease, characterised by decreased bone mass per unit volume and microarchitectural deterioration of bone tissue with porous formations being formed in the bones, leading to a decrease in the quantity of calcium in bones and a high risk of fracture for any bone, including the hip.

It is known from the prior art that a great number of preparations, which are mostly imported, are used for the prevention and treatment of osteoporosis. They can be subdivided into three broad groups:

Preparations that inhibit bone resorption:

- estrogenic hormones (Divina, Divigel, Divitren, Estrofem, Climen, Klimonorm, tibolone etc.),
- calcitonin,
- bisphosphonates (alendronate, chlodronate, Aclasta etc.),
- calcium preparations,
- vitamin D (calciferol, cholecalciferol),
- active metabolites of vitamin D (calcitriol, alphacalcitriol,)
- thiazide diuretics,
- ossein-hydroxyapatite,
- anabolic steroids (oxandrolone, nandrolone, stanozolol).

Preparations that increase bone mass:

- fluorine derivatives (sodium fluoride, monofluorophosphate),
- anabolic steroids,
- ipriflavones,
- ossein-hydroxyapatite,
- peptide (1-34) PTH,
- prostaglandin E2,
- growth hormone,
- parathyroid hormone.

Agents that influence both these processes:

- vitamin D: cholecalciferol (D3), ergocalciferol (D2),
- active metabolites of vitamin D: calcitriol, Alphadol (Oksidevit, Alpha D3-Teva);
- a combination of calcium and vitamin D.

The preparations in these groups are widely used as sole treatment or as part of combined treatment (Ideos, Kaltsii D3 NIKOMED, Alphadol calcium, calcium carbonate, calcium citrate, etc.).

Therefore, as hundreds of preparations are currently available, it can be difficult for a medical practitioner to select an effective bone protector to treat osteoporosis. Well-placed advertisements for these preparations only make it more difficult to choose a suitable preparation. Therefore, it is important to develop methods for diagnosing osteoporosis and means of measuring the effectiveness of a preparation in treating the disease.

Laboratory, histological and radiological methods are currently used to diagnose OP and measure the effectiveness of the therapeutic activity of a preparation. A histomorphological analysis of biopsy material of the iliac crest with an assessment of osteoclast and osteoblast activity was previously considered to be the most reliable method of diagnosing OP. However, the invasive nature of these examinations limits their use. For this reason, radiological methods are currently used to diagnose OP and consequently to monitor treatment.

Standard radiography is one of the essential methods of examination. It makes it possible to carry out a morphometric analysis of vertebral bodies, identify characteristic deformities in vertebral bodies, and identify fractures in vertebral bodies with high accuracy. A standard roentgenogram is the primary and most accessible method of diagnosing osteoporosis. However, this diagnostic method relates to late diagnostic methods, as it allows specialists to detect osteoporosis only when there has been a loss of over 30-40% of bone mass. So radiography is unsuitable for early quantitative diagnosis of osteopenia, osteoporosis, and monitoring therapy.

The most accurate and informative method of examining mineral density of the bone tissue (BMD) is radiographic absorptiometry. The company "Osteometer" has developed a series of instruments for measuring BMD in the distal forearm. The DTX-100 and DTX-200 apparatuses have special software for calculating the annual loss of bone tissue, and make it possible to predict the risk of bone fractures. Modern osteometers allow us to measure BMD in various parts of the body, including the hip and vertebral bodies. All osteometers operate on two scoring scales.

The T-score indicates the number of standard deviations above or below the mean peak bone mass. The T-score decreases in parallel with the gradual decrease of bone mass with increasing age, and is used to measure BMD in adults.

The Z-score indicates the number of standard deviations above or below the mean for individuals of the same age. BMD is measured in absolute units, in standard deviations between the BMD of the patient and the age-matched mean of healthy children and adolescents of the same age and sex.

It is known from the prior art that measuring the severity of osteoporosis and the effectiveness of treatment is carried out by evaluating the results of a BMD examination, according to the classification of the WHO:

BMD normal mean – indicating a T-score from + 1 to – 1 standard deviation (SD) from peak bone mass.

I degree osteopenia – BMD from -1 to -1.5 SD.

II degree osteopenia – BMD from -1.5 to -2.0 SD.

III degree osteopenia – BMD from -2.0 to -2.5 SD.

I degree osteoporosis – BMD from -2.5 and less without fractures.

II degree osteoporosis – BMD from – 2.5 and less where osteoporotic bone fractures are present.

These scores are currently used for diagnosis and to measure the effectiveness of treatment with a particular preparation.

Disadvantage of the known method:

This method only takes into account the quantitative characteristic of bone mineralisation which, depending on the body mass, region, sex, and skin colour of the patient, can significantly deviate from standard values. For example, women with low body mass generally have lower BMD values than women who weigh more, particularly if they are obese, which makes it difficult to quantitatively characterise the changes caused by osteoporosis. However, it is not possible to perform an objective qualitative evaluation of the effectiveness of a preparation in treating osteoporosis on the basis of increased or decreased BMD. This is compounded by the lack of regional databases on standard regional BMD.

It is not known from the prior art, and the authors have ascertained through studies that the degree of severity of osteoporosis, changes therein and the measurement of the effectiveness of a preparation must be calculated on the basis of qualitative criteria: on the basis of the morphology of osteoporotic manifestations and specifically, the measurement of cavities in trabecular bone sections. It is difficult or impossible to determine the severity of the disease and the effectiveness of a given preparation without taking the morphometric manifestations (cavities) of osteoporosis into consideration.

The problem being addressed by the claimed invention is to objectively evaluate the effectiveness of a preparation (preparations) (bone protector) and measure cavities in trabecular bone sections. It proposes to improve the diagnostic capabilities of computerised osteometers by measuring cavities caused by osteoporosis and determine the severity of the disease not only on the basis of mineral density, but also on the basis of the presence of cavities in trabecular bone sections.

This problem is solved in that in the method of diagnosing osteoporosis, including laboratory, histological and radiological methods of examination (standard radiography) and the measurement of bone mineral density using the radiological absorptive method on osteometers, into which an additional diagnostic criterion is introduced, namely the measurement of cavities by adjusting them specially to measure cavities in trabecular bone sections using the radiological absorptive method and when diagnosing osteoporosis on osteometers, distilled water is introduced into special incubation apparatuses around the examined limb bones of the hands and feet.

A list of osteometric studies of patients observed by the authors is given below. These patients had not been diagnosed with osteoporosis based on the results of BMD measurements (see examples)

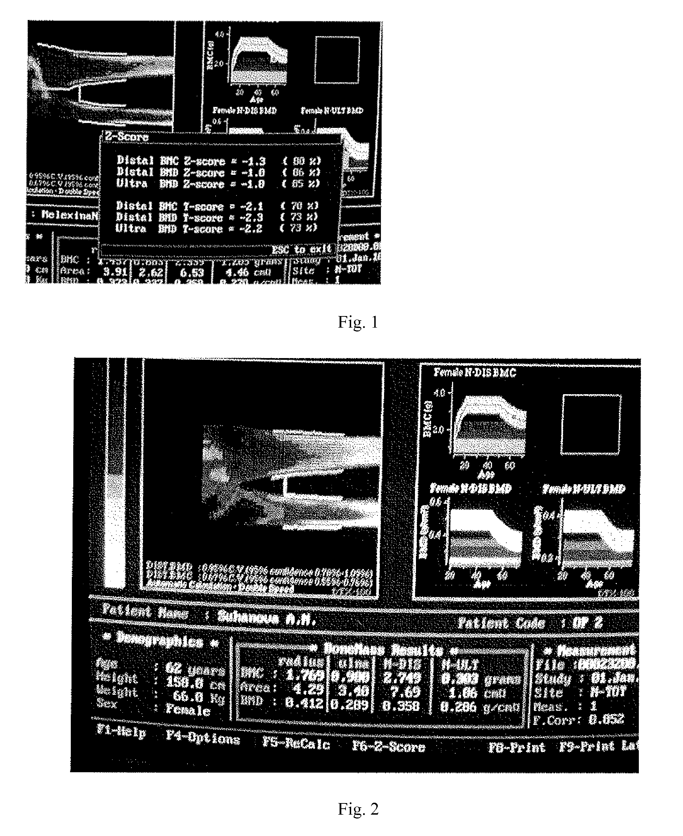

*Example 1.* Female patient A. Decrease in BMD T-score from -2.1 to -2.3 SD. Under the WHO classification, the diagnosis would be 3 degree osteopenia. However, there is a cavity measuring 2x4 mm in the trabecular section of the ulna of the patient. This is the principal indicator of two indicators - it is a sign of osteoporosis. (see Fig 1.)

*Example 2.* The BMD (bone mineral density) of female patient B corresponds to 2 degree osteopenia (-1.8 SD). However, in the trabecular bone sections there are sections with significant demineralisation, which the authors refer to as "cavities", i.e., this is osteoporosis, not osteopenia. (see Fig 2).

It is not known from the prior art that detecting cavities in bones or negative changes in values for bone mineral density is significant in diagnosing osteoporosis. However all modern osteometers do not "see" cavities, as they are only set to measure BMD, which is a disadvantage of such apparatuses. In order for bone cavities to be visible, it is necessary to create osteometers which comply with certain conditions. The authors have established that there must be distilled water in special incubation apparatuses around the limb bones (arm, leg) being examined. Then it is possible for osteometers such as the DTX-100 to measure BMD and cavities simultaneously. If there is air surrounding the organs being examined instead of distilled water, cavities will not be measured. The experience of the authors and new knowledge about osteoporosis shows that there is now a great need for such apparatuses, as they also measure two additional parameters: cavities and excess salt deposits in soft tissues. For other types of osteometers (such as the DTX-200), special settings are required in order for the apparatus to "see" cavities, these settings were created by the authors on the basis of the reference data recorded on a DTX-100 apparatus.

In certain situations when operating one of these "seeing" apparatuses the BMD indicators are decreased, for example to -2.4 SD (osteopenia), however when cavities are discovered in the bones of such patients, they must be diagnosed with osteoporosis. This is particularly important in cases where these cavities increase during observation or bone fractures are indicated in the medical history of the patient.

For this reason, measuring the effectiveness of a preparation for treating osteoporosis on next generation apparatuses such as the DTX-200 (which does not see cavities) on the basis of only one indicator- BMD - may be inexact.

For example, female patient F was prescribed Kaltsii D3 NIKOMED. Example 2 shows the results after 12 months of treatment. Mineralisation increased from -3.4 to -1.9 SD (from osteoporosis to 2 degree osteopenia), but cavities increased. Thus if the effectiveness of the treatment is evaluated on a DTX 200 apparatus on the basis of BMD, this may be evaluated as a positive result, and consequently, the effectiveness of this preparation (Kaltsii D3 NIKOMED) and the possibility of withdrawing the preparation or altering its dose, reducing the duration of treatment etc.

If changes in the disease and the effectiveness of the preparation "Kaltsii D3 NIKOMED" in treating osteoporosis are measured on a DTX-100 apparatus, (or a DTX-200 with special settings, which "sees" cavities and simultaneously measures bone mineral density), the conclusion drawn from such instrument readings will be completely different — an increase in the severity of the osteoporosis, due to an increase in the dimensions of cavities, with some underlying improvement in bone mineralisation. The preparation "Kaltsii D3 NIKOMED" is not effective. The preparation must be replaced by another, because of the increase in cavities, i.e. the absence of an effect in restoring the bone tissue structure.

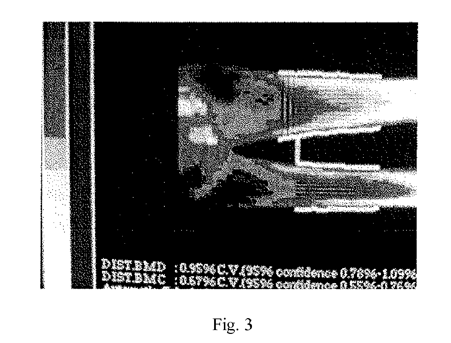

Example 3. Female patient F. Post-menopausal osteoporosis. Cavities in metaphyseal (trabecular) bone sections, BMD T-score - 3.4 SD. (see Fig 3)

The aim of this invention is to develop a method to improve diagnosis of osteoporosis and to objectively evaluate the effectiveness of the preparation (s) (bone protector).
The authors propose the following method:

Include a homogenous group of patients of the same age and sex in the test group and the comparison group.

1. Select patients to measure the effectiveness of the preparation: only with cavities present;

2. Patients with BMD of -2.5 SD and lower

3. Only female patients with primary osteoporosis (for example, with post-menopausal osteoporosis). Or only male patients with primary osteoporosis.

4. Exclude patients with secondary osteoporosis with various systemic endocrine diseases, oncopathologies etc.

5. Patients give their consent to participate in the experiment.

6. Patients must not know which preparation they are taking (blind test)

7. Radiological absorptive osteometry is conducted on both groups before treatment begins and every three-four months thereafter.

8. The results achieved are subject to standard statistical processing. Any preparation may be selected as a reference (control), preferably the most widely purchased.

**Claims**

1. A method for diagnosing osteoporosis, which involves laboratory, histological, radiological methods of examination (standard radiography) and measuring bone mineral density using the radiological absorptive method.

2. The method according to claim 1 is **characterised in that** the diagnosis is carried out on osteometers.

3. The method according to claim 1 is **characterised in that** an additional diagnostic criterion is introduced into the osteometers , namely the measurement of cavities by specially adjusting them to measure cavities in trabecular bone sections using the radiological absorptive method.

4. The method according to claim 1 is **characterised in that** when diagnosing osteoporosis on osteometers, distilled water is introduced into special incubation apparatuses around the limb bones of the hands and feet which are being examined.

Fig. 1

Fig. 2

Fig. 3

## INTERNATIONAL SEARCH REPORT

| International application No. |
| --- |
| PCT/RU 2012/000688 |

**A. CLASSIFICATION OF SUBJECT MATTER**
A61B 6/00 (2006.01); G01N 9/36 (2006.01); G01N 23/083 (2006.01)

According to International Patent Classification (IPC) or to both national classification and IPC

**B. FIELDS SEARCHED**

Minimum documentation searched (classification system followed by classification symbols)
A61B 6/00, G01N 9/36, 23/083

Documentation searched other than minimum documentation to the extent that such documents are included in the fields searched

Electronic data base consulted during the international search (name of data base and, where practicable, search terms used)

CIPO, DEPATISnet, DWPI, EAPATIS, Esp@ce, Esp@cenet, KIPRIS, MEDLINE, PAJ, PCT Online, PatSearch, REGISTRY, RSL, RUPTO, Rambler, SIPO, WIPO, Yandex, e-library, BEN (Benran), VINITI, RMZH, ROSPATENT

**C. DOCUMENTS CONSIDERED TO BE RELEVANT**

| Category* | Citation of document, with indication, where appropriate, of the relevant passages | Relevant to claim No. |
| --- | --- | --- |
| X<br>Y | BARTL S. et al. Secondary osteoporosis: pathogenesis, types, diagnostics and therapy. Radiologe, 2011 Apr; 51(4):307-24, the abstract, [on-line], [retrieved on 24.12.2012]. Found from PubMed, PMID:21455799 | 1<br>2-4 |
| Y | STRUKOV V.I. et al. Kliniko-metabolicheskie osobennosti rakhita u detei, rodivshikhsya ot materei s osteopenicheskim sindromom. Pediatriya, 2004, N. 5, p. 24-25 | 2 |
| Y | RU 2305491 C2 (ROSSYSKAYA MEDITSINSKAYA AKADEMIYA POSLEDIPLOMNOGO OBRAZOVANIYA MINISTERSTVA ZDRAVOOKHRANENIYA ROSSYSKOI FEDERATSII) 10.09.2007, the abstract, fig. 1, p. 4, par. 4-7 - p. 5, par. 1-4, p. 8, par. 6-8 - p. 9, p. 10, par. 1-3 | 3 |
| Y | RU 2412650 C1 (ZAKRYTOE AKTSIONERNOE OBSCHESTVO "RENTGENPROM") 27.02.2011, p. 3, par. 6-7 | 4 |

☒ Further documents are listed in the continuation of Box C.      ☐ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 21 December 2012 (13.12.2012) | 24 January 2013 (24.01.2013) |

| Name and mailing address of the ISA/<br>RU | Authorized officer |
| --- | --- |
| Facsimile No. | Telephone No. |

Form PCT/ISA/210 (second sheet) (April 2005)

## INTERNATIONAL SEARCH REPORT

International application No.

PCT/RU 2012/000688

| C (Continuation). | DOCUMENTS CONSIDERED TO BE RELEVANT | |
|---|---|---|
| Category* | Citation of document, with indication, where appropriate, of the relevant passages | Relevant to claim No. |
| A | RU 2159577 C1 (MIKHAILOV MARS KONSTANTINOVICH et al.) 27.11.2000 | 1-4 |

Form PCT/ISA/210 (continuation of second sheet) (April 2005)